# EUROPEAN PATENT APPLICATION

(11) **EP 2 746 391 A1**
(43) Date of publication of application: **25.06.2014**
(21) Application number: 12198007.2
(22) Date of filing: 19.12.2012
(51) Int. Cl.: C12N 9/50, C12N 15/62, C12P 21/06

(54) **Method for producing a recombinant protein of interest**

(71) Applicant: SANDOZ AG, 4056 Basel (CH); Boehringer Ingelheim RCV GmbH & Co KG, 1121 Wien (AT)
(72) Inventor: Sponring, Michael, 6123 Terfens (AT); Schneider, Rainer, 6300 Wörgl (AT); Auer, Bernhard, 6020 Innsbruck (AT)
(74) Representative: Sonn & Partner Patentanwälte

(57) **Abstract**

Disclosed is a method for producing a recombinant protein of interest which is characterised by the following steps:
(a) providing a first part of an N^{pro} autoprotease and providing a second part of an N^{pro} autoprotease, wherein said second part is fused by a peptidic bond to a protein of interest but said second part alone does not exhibit a proteolytic activity, and wherein complementation of said first part with the second part forms an autoproteolytically active N^{pro} autoprotease,
(b) contacting the first part of the N^{pro} autoprotease with the second part of the N^{pro} autoprotease so that an autoproteolytically active N^{pro} autoprotease is formed and the protein of interest fused by the peptidic bond to the second part of the N^{pro} autoprotease is proteolytically cleaved off the second part of the N^{pro} autoprotease at the peptidic bond, and
(c) recovering the protein of interest.

SEQUENCE LISTING

<110>  Sandoz AG
       Boehringer Ingelheim RCV GmbH & Co KG

<120>  Method for producing a recombinant protein of interest

<130>  R 61680

<160>  13

<170>  PatentIn version 3.5

<210>  1
<211>  92
<212>  PRT
<213>  Artificial Sequence

<220>
<223>  CSFV-derived

<400>  1

Met Glu Pro Val Tyr Asp Thr Ala Gly Arg Pro Leu Phe Gly Asn Pro
1               5                   10                  15

Ser Glu Val His Pro Gln Ser Thr Leu Lys Leu Pro His Asp Arg Gly
            20                  25                  30

Arg Gly Asp Ile Arg Thr Thr Leu Arg Asp Leu Pro Arg Lys Gly Asp
        35                  40                  45

Cys Arg Ser Gly Asn His Leu Gly Pro Val Ser Gly Ile Tyr Ile Lys
    50                  55                  60

Pro Gly Pro Val Tyr Tyr Gln Asp Tyr Thr Gly Pro Val Tyr His Arg
65                  70                  75                  80

Ala Pro Leu Glu Phe Phe His His His His His His
                85                  90

<210>  2
<211>  72
<212>  PRT
<213>  Artificial Sequence

<220>
<223>  CSFV-derived

<400>  2

Met His His His His His His Glu Thr Thr Lys Arg Ile Gly Arg Val
1               5                   10                  15

Thr Gly Ser Asp Gly Lys Leu Tyr His Ile Tyr Val Glu Val Asp Gly
            20                  25                  30

Glu Ile Leu Leu Lys Leu Ala Lys Arg Gly Thr Pro Arg Thr Leu Lys
        35                  40                  45

Trp Thr Arg Asn Thr Thr Asn Cys Pro Leu Trp Val Thr Ser Cys Ser
    50                  55                  60

Trp Ser His Pro Gln Phe Glu Lys
65                  70

<210>  3
<211>  66
<212>  PRT
<213>  Artificial Sequence

<220>
<223>  CSFV-derived

<400>  3

Met Glu Thr Thr Lys Arg Ile Gly Arg Val Thr Gly Ser Asp Gly Lys
1               5                   10                  15

Leu Tyr His Ile Tyr Val Glu Val Asp Gly Glu Ile Leu Leu Lys Leu
            20                  25                  30

Ala Lys Arg Gly Thr Pro Arg Thr Leu Lys Trp Thr Arg Asn Thr Thr
        35                  40                  45

Asn Cys Pro Leu Trp Val Thr Ser Cys Ser Trp Ser His Pro Gln Phe
    50                  55                  60

Glu Lys
65

<210>  4
<211>  73
<212>  PRT
<213>  Artificial Sequence

<220>
<223>  CSFV-derived

<400>  4

Met Glu Thr Thr Lys Arg Ile Gly Arg Val Thr Gly Ser Asp Gly Lys
1               5                   10                  15

Leu Tyr His Ile Tyr Val Glu Val Asp Gly Glu Ile Leu Leu Lys Leu
            20                  25                  30

Ala Lys Arg Gly Thr Pro Arg Thr Leu Lys Trp Thr Arg Asn Thr Thr
        35                  40                  45

Asn Cys Pro Leu Trp Val Thr Ser Cys Ser Val Asp Lys Leu Ala Ala

50                  55                  60

Ala Leu Glu His His His His His His
65                  70

<210>  5
<211>  214
<212>  PRT
<213>  Artificial Sequence

<220>
<223>  CSFV-derived

<400>  5

Met Glu Thr Thr Lys Arg Ile Gly Arg Val Thr Gly Ser Asp Gly Lys
1               5                   10                  15

Leu Tyr His Ile Tyr Val Glu Val Asp Gly Glu Ile Leu Leu Lys Leu
            20                  25                  30

Ala Lys Arg Gly Thr Pro Arg Thr Leu Lys Trp Thr Arg Asn Thr Thr
        35                  40                  45

Asn Cys Pro Leu Trp Val Thr Ser Cys Met Ala Arg Val Gln Phe Lys
    50                  55                  60

Gln Arg Glu Ser Thr Asp Ala Ile Phe Val His Cys Ser Ala Thr Lys
65                  70                  75                  80

Pro Ser Gln Asn Val Gly Val Arg Glu Ile Arg Gln Trp His Lys Glu
                85                  90                  95

Gln Gly Trp Leu Asp Val Gly Tyr His Phe Ile Ile Lys Arg Asp Gly
            100                 105                 110

Thr Val Glu Ala Gly Arg Asp Glu Met Ala Val Gly Ser His Ala Lys
        115                 120                 125

Gly Tyr Asn His Asn Ser Ile Gly Val Cys Leu Val Gly Gly Ile Asp
    130                 135                 140

Asp Lys Gly Lys Phe Asp Ala Asn Phe Thr Pro Ala Gln Met Gln Ser
145                 150                 155                 160

Leu Arg Ser Leu Leu Val Thr Leu Leu Ala Lys Tyr Glu Gly Ala Gly
                165                 170                 175

Leu Arg Ala His His Glu Val Ala Pro Lys Ala Cys Pro Ser Phe Asp
            180                 185                 190

Leu Lys Arg Trp Trp Glu Lys Asn Glu Leu Val Thr Ser Asp Arg Gly
        195                 200                 205

His His His His His His
    210

<210>  6
<211>  24
<212>  DNA
<213>  Artificial Sequence

<220>
<223>  CSFV-derived

<400>  6
gaaattaata cgactcacta tagg                                              24

<210>  7
<211>  32
<212>  DNA
<213>  Artificial Sequence

<220>
<223>  CSFV-derived

<400>  7
atatagtcga cttattaaaa gaattccaga gg                                     32

<210>  8
<211>  48
<212>  DNA
<213>  Artificial Sequence

<220>
<223>  CSFV-derived

<400>  8
atagtcgact tattagtgat ggtgatggtg atgaaagaat tccagagg                    48

<210>  9
<211>  31
<212>  DNA
<213>  Artificial Sequence

<220>
<223>  CSFV-derived

<400>  9
tatacatatg gaaaccacta aacgtattgg c                                      31

<210>  10
<211>  49
<212>  DNA
<213>  Artificial Sequence

<220>
<223>  CSFV-derived

<400>  10

tatacatatg catcaccatc accatcacga aaccactaaa cgtattggc                49

<210>  11
<211>  24
<212>  DNA
<213>  Artificial Sequence

<220>
<223>  CSFV-derived

<400>  11
gttatgctag ttattgctca gcgg                                              24

<210>  12
<211>  16
<212>  PRT
<213>  Artificial Sequence

<220>
<223>  model peptide pep6His

<400>  12

Ser Val Asp Lys Leu Ala Ala Ala Leu Glu His His His His His His
1               5                   10                  15

<210>  13
<211>  148
<212>  PRT
<213>  artificial sequence

<220>
<223>  HoBi-Variant

<400>  13

Met Glu Pro Leu Tyr Asp Lys Asn Gly Ala Val Leu Phe Gly Glu Pro
1               5                   10                  15

Ser Asp Thr His Pro Gln Ser Thr Leu Lys Leu Pro His Pro Arg Gly
            20                  25                  30

Glu Lys Glu Val Ile Val Gly Ile Arg Asp Leu Pro Arg Lys Gly Asp
        35                  40                  45

Cys Arg Thr Gly Asn Arg Leu Gly Pro Val Ser Gly Leu Phe Val Lys
    50                  55                  60

Pro Gly Pro Val Phe Tyr Gln Asp Tyr Ser Gly Pro Val Tyr His Arg
65                  70                  75                  80

Ala Pro Leu Glu Gln Phe Lys Gln Ala Pro Met Cys Glu Val Thr Lys
                85                  90                  95

Arg Ile Gly Arg Val Thr Gly Ser Asp Gly Asn Leu Tyr His Met Tyr
            100                 105                 110

Val Cys Thr Asp Gly Cys Ile Leu Val Lys Thr Ala Lys Arg Glu Gly
        115                 120                 125

Gln Asp Val Leu Lys Trp Val Tyr Asn Val Leu Asp Ser Pro Ile Trp
    130                 135                 140

Val Thr Ser Cys
145

## Description

The present invention relates to a process for the recombinant production of a desired heterologous polypeptide of interest by using the autoprotease Npro of Pestivirus-technology.

Inteins (INTernal protEINS) constitute protein sequences surrounded by exteins. Inteins catalyse protein splicing without any additional cofactors. In a first step a thioester bond or an ester bond is formed at the N-terminal splicing junction by an acyl shift of a cysteine or serine residue. A transesterification connects the N-terminal extein with the C-terminal extein. Cyclization of an asparagine at the C-terminal splice site results in a free intein and in thio(ester) linked exteins. A second acyl shift yields a natural peptide bond between the N-terminal and C-terminal extein.

Cis splicing as well as trans splicing inteins have been found in all domains of life. Trans acting inteins are split into an N-terminal and a C-terminal fragment. After reassembling of both fragments an active intein is formed. This principle offers many possibilities in biotechnological applications. Inteins can be used for protein purification, protein backbone cyclization, labelling proteins, array construction, and introduction of unnatural substitutions.

One application of inteins is in the field of antimicrobial drug development by inhibition of intein splicing. Inteins are generally found interrupting the catalytic core of essential proteins. However, there are no known inteins in animals or humans. 60 different molecules were identified with low IC50 values, which inhibit Mtu recA and dnaB inteins in an effort to conquer Mycobacterium tuberculosis. However, it has not been proven that the antimicrobial activity is by inhibition of inteins and not by other effects like alkylation of active site cysteins.

Intein based cyclization of therapeutic peptides makes them resistant to exopeptidases and reduces their entropy. Polypeptides can be cyclized in vivo as well as in vitro. The DnaE split intein from Synechocystis sp PCC6803 (Ssp DnaE split intein) was used to cyclize the eight amino acid long tyrosinase inhibitor pseudostellarin F in vivo and by doing so enhancing its production rate. Furthermore, by cyclization of the E. coli dihydrofolate reductase its thermostability could be improved. In vitro experiments used the Ssp DnaE split intein to mediate splicing as well as peptide bond cleavage. In addition trans splicing was accomplished on column using a chitin resin for ligation of two proteins under native conditions. Inhibitors of E. coli Dam methyltransferase were identified using a method named Split Intein Circular Ligation of Peptides and Proteins (SICLOPPS). The IC50 values of the cyclized peptides were much lower than that of the linear ones and in the same range as the naturally occurring inhibitor sinefungin.

Protein purification using inteins is useful because there is no need for a protease to cleave off a fusion protein or an affinity tag. Intein mediated Expressed Protein Ligation (EPL) was successfully used to generate doubly phosphorylated Serotonin N-acetyltransferase (AANAT). This changes its catalytic activity and stability. Furthermore, using this protein semisynthesis method fluorescently labelled 14-3-3ζ was generated which was then used to quantify the binding of mono- and diphosphorylated AANAT by anisotropy. More protein modifications using EPL include introduction of selenocysteine, unnatural amino acids, FRET pairs, biotin or other tags, membrane-protein anchors, DNA, and photo-activated compounds.

The split intein Ssp GyrB S11 can be divided into an extremely small (6 aa) C-intein and a large (150 aa) N-intein [Volkmann 2008]. This system can be used for C-terminal labelling of recombinant proteins with modified peptides.

An intein based biosensor for measuring the activity of the caspase-3 was developed using the Ssp DnaE split intein. Firefly luciferase (Luc) was connected with caspase-3 substrates. After cyclization of Luc its function was abolished until a protease generated a linear Luc protein.

Another application for inteins is in the field of in vivo control of protein function. A thyroid hormone binding domain was inserted into an intein and hence abolishing its splicing activity. By addition of human thyroid hormone or analogues the intein gained its functions back and the active intein was used to activate a variety of different proteins in E. coli in a dose depend manner.

The S. cerevisiae VMA intein (vacuolar ATPase subunit) was used as a temperature sensitive switch to control the activation of the transcription regulators Gal4 and Gal80. The Gal80-intein was able to regulate the Gal4 upstream activation sequence temperature dependent in D. melanogaster.

Trans splicing can also be a valuable tool in gene therapy. Coagulation factor VIII (FVIII) is secreted as a heterodimer consisting of a heavy chain (HC) and a light chain (LC) which is secreted much more efficient than the HC. The results of these trans splicing experiments suggest that the FVIII LCs are critical in maintaining the proper configuration of HCs for secretion.

Trans splicing using the Ssp DnaE intein can be conducted in plants as well. The N-terminal fragments of 5-enol-pyruvylshikimate-3-phosphate synthase (EPSPS) fused to the N-terminal part of Ssp DnaE was integrated into the nuclear DNA of N. tabacum. The remaining EPSPS gene fragment fused to the C-terminal part of Ssp DnaE was integrated into the chloroplast genome. Trans splicing utilized full length EPSPS and improved resistance to the herbicide N-phosphonomethyl-glycine (glyphosphate).

Drawbacks of intein based technologies are spontaneous cleavages during protein expression and purification (Volkmann et al. (Protein Science 18 (2009), 2393-2402). This significantly decreases the yield of the desired protein product and complicates subsequent uses of the cleaved protein. Another issue is the specificity of inteins. A screening using SICLOPSS showed that less than 70% of randomly selected clones showed detectably levels of in vivo cyclization (Cheriyan et al., Adv.Drug Del. Rev. 61 (2009), 899-907).

It is an object of the present invention to provide an improved alternative to intein technology for producing recombinant proteins, especially proteins for therapeutic use in humans. The method should be easily controllable and suitable for up-scaling to provide industrial production of such proteins.

Therefore, the present invention provides a method for producing a recombinant protein of interest which is characterised by the following steps:
(a) providing a first part of an N^{pro} autoprotease and providing a second part of an N^{pro} autoprotease, wherein said second part is fused by a peptidic bond to a protein of interest but said second part alone does not exhibit a proteolytic activity, and wherein complementation of said first part with the second part forms an autoproteolytically active N^{pro} autoprotease,
(b) contacting the first part of the N^{pro} autoprotease with the second part of the N^{pro} autoprotease so that an autoproteolytically active N^{pro} autoprotease is formed and the protein of interest fused by the peptidic bond to the second part of the N^{pro} autoprotease is proteolytically cleaved off the second part of the N^{pro} autoprotease at the peptidic bond, and
(c) recovering the protein of interest.

The present invention is an improvement in the recombinant production of a desired heterologous polypeptide of interest by using the autoprotease N^{pro} of Pestivirus-technology. This technology usually provides the recombinant expression of a fusion polypeptide which comprises an autoproteolytic moiety directly or indirectly derived from autoprotease N^{pro} of Pestivirus and a heterologous polypeptide of interest in a host cell, often a prokaryotic host cell, such as E. coli. The heterologous polypeptide or protein of interest is covalently coupled via a peptide bond to the N^{pro} molecule. The protein of interest is released from the fusion protein through hydrolysis of the peptide bond between the C-terminal Cys168 of Npro and position 169 of the fusion polypeptide which represents the authentic N-terminal amino acid of the protein of interest to be produced according to the present invention. The heterologous polypeptide of interest is produced in the host cell in form of cytoplasmic inclusion bodies (IB), which are then isolated and treated in such a way, that the desired heterologous polypeptide is cleaved from the fusion polypeptide by the N^{pro} autoproteolytic activity. With the present invention, the metabolic burden of the host cell can be significantly reduced because the first part of the N^{pro} autoprotease does not have to be expressed together with the fusion polypeptide comprising the protein of interest (it can be produced separately). This leads to significantly higher yields and shorter production times.

Fusion polypeptides comprising the autoprotease Npro of Pestivirus are therefore specifically useful for producing heterologous recombinant polypeptides. N^{pro} is an autoprotease with length of 168 amino acids and an apparent Mᵣ of about 20 kD in vivo. It is the first protein in the polyprotein of Pestiviruses and undergoes autoproteolytic cleavage from the following nucleocapsid protein C. This cleavage takes place after the last amino acid in the sequence of N^{pro}, Cys168. The autoprotease N^{pro} activity of Pestivirus always cleaves off the fusion partner at this clearly determined site, releasing a polypeptide of interest with homogenous N-terminus. In addition, the autoproteolytic activity of N^{pro} can be induced in vitro, by application of special buffers, so that the polypeptide of interest can be obtained by cleavage of fusion polypeptides that are expressed in IBs.

N-terminal autoprotease N^{pro} from Classical Swine Fever Virus (CSFV) used in this technology serves as an attractive tool for expression of therapeutic proteins in large amounts especially in E. coli. Medical applications require an authentic N-terminus of the recombinant proteins, which can be achieved by self-cleavage of N-terminally fused N^{pro} autoprotease. In addition, N^{pro} fusion technology also allows the expression of small or toxic peptides, which would be degraded immediately after their synthesis by host cell proteases (Achmüller et al., Nat. Methods 4 (2007), 1037-1043). As the expression of N^{pro} fusion proteins in E. coli leads to the formation of insoluble aggregates, known as inclusion bodies, appropriate resolving and renaturation protocols are required to obtain biological active proteins.

As already mentioned, in most cases solubilisation is carried out in chaotropic agents such as urea or guanidinium chloride at high concentrations in combination with reducing agents to abolish false formed disulfide bonds. Due to its simplicity refolding by dilution is widely used to initiate renaturation.

The present invention is carried out with the N^{pro} technology. This technology is disclosed e.g. in WO 01/11057 A, WO 01/11056 A, WO 2006/113957 A, WO 2006/113958 A, WO 2006/113959 A, and Achmüller et al., Nat. Meth. 4 (2007), 1037-1043. In general terms, the N^{pro} technology relates to a process for the recombinant production of a heterologous protein of interest, comprising (i) cultivation of a bacterial host cell which is transformed with an expression vector which comprises a nucleic acid molecule which codes for a fusion protein, the fusion protein comprising a first polypeptide which exhibits the autoproteolytic function of an autoprotease N^{pro} of a Pestivirus, and a second polypeptide which is connected to the first polypeptide at the C-terminus of the first polypeptide in a manner such that the second polypeptide is capable of being cleaved from the fusion protein by the autoproteolytic activity of the first polypeptide, and the second polypeptide being a heterologous protein of interest, wherein cultivation occurs under conditions which cause expression of the fusion protein and formation of corresponding cytoplasmic inclusion bodies, (ii) isolation of the inclusion bodies from the host cell, (iii) solubilisation of the isolated inclusion bodies, (iv) dilution of the solubilisate to give a reaction solution in which the autoproteolytic cleavage of the heterologous protein of interest from the fusion protein is performed (the present invention specifically aims on this step by providing an alternative embodiment to this step), and (v) isolation of the cleaved heterologous protein of interest ("recovering"). According to the present invention, the first polypeptide, the N^{pro} autoprotease, is provided in a cleaved status, the method according to the present invention establishing (auto)proteolytic activity by complementation of the first and second part of the first polypeptide (therefore, the "auto"proteolytic activity is an activity of the re-combined N^{pro} molecule).

The term "denatured form" (or "non-refolded form") in the meaning of the present invention designates the biologically inactive form of the expressed fusion protein comprising the second part of the N^{pro} autoprotease as well as the biologically inactive form of the first part of the N^{pro} autoprotease, as obtained as a product of the recombinant production process, usually as obtained after solubilising the inclusion bodies (under conditions under which the native three dimensional structure of the fusion protein (as well as of the first part of the N^{pro} autoprotease) is disrupted).

The term "refolding" (or "renaturing") refers to the mechanism during which the solubilized protein or part of protein regains its native conformation and biological activity (or the biological activity of the part, respectively), i.e. reconstituting a protein from its denatured, inactive state to its active form (see also: Kaar et al., Biotech. Bioeng. 104 (2009), 774-784).

The term "autoproteolytic function", "autoproteolytic cleavage" or "autoproteolytic" refers to the proteolytic activity of the complementation of the first part with the second part of the autoprotease, wherein the second part is fused to the protein of interest (insofar, the term "autoprotease" according to the present invention is used in a different way as usual, because the proteolytic activity is not drawn to the protein having the proteolytic activity, but drawn to the complementation of the first part of the N^{pro} autoprotease and the second part of the N^{pro} autoprotease (the latter including the protein of interest); however, the two parts were generated from a "classical" autoprotease). As used herein the term "autoprotease" shall therefore refer to the complemented first and second part of a polypeptide that possesses proteolytic activity and is capable of cleaving the other (second) part of the autoprotease from the protein of interest, i.e. the complemented N^{pro} autoprotease cleaves (via its proteolytic activity generated by the complementation step) its second part from the fusion protein (the fusion protein comprising the second part of the autoprotease fused to the protein of interest). The first part of the N^{pro} autoprotease may have already proteolytic activity; the fusion protein comprising the second part of the N^{pro} autoprotease must not have autoproteolytic activity (so that cleavage of the protein of interest from the second part of the Npro autoprotease cannot occur until complementation with the first part of the N^{pro} autoprotease). Accordingly, complementation of said first part with the second part can be effected to provide an autoproteolytically active N^{pro} autoprotease. This complementation can be directed by appropriate renaturing conditions which do not only allow spatial coordination of the two parts of the N^{pro} autoprotease, but also functional restoration of the autoproteolytic activity for effecting the cleavage event.

This cleavage event can be directed by various embodiments according to the present invention. There are also various possibilities for providing the first part of the N^{pro} autoprotease and the fusion protein comprising the second part of the N^{pro} autoprotease. For example, the first part of the N^{pro} autoprotease can be expressed/produced by fermentation solely. Alternatively or additionally, the second part of the N^{pro} autoprotease (as fusion protein) can be expressed/produced by fermentation solely. Further embodiments include the possibility to provide the first part of the N^{pro} autoprotease and/or the second of the N^{pro} autoprotease (as fusion protein) as inclusion bodies (IB); the two polypeptides (or only one of them) can be provided as soluble forms in cytoplasm (preferably the shorter polypeptide (usually the first part). Also combinations are possible (e.g. to provide the first part soluble and second part as IB (or the first part as IB and second soluble). The cleavage event can be diligently directed within the rationale of the present invention. For example, a denaturated first part can be mixed with a denaturated second part, refolded together and effect cleavage; the first part may also be refolded separately from the second part, mixed with the (also separately refolded) second part and allowed to cleave. It is also possible, e.g. to refold the first part, mix it with the denatured second part, refold the second part and effect cleavage. Another preferred embodiment comprises the provision of the first part in denaturated form and the second part in refolded form, mix the polypeptides, refold the first part and allow cleavage.

The autoproteolytic cleavage rate can be determined, for example, by initially solubilising the isolated/purified inclusion bodies (with the expressed fusion protein comprising the second part of the autoprotease fused to the protein of interest) in 7 M guanidine/HCI solution and then diluting 1:100 in reaction solution and adding the first part of the autoprotease to initiate proteolytic activity of the complemented autoprotease. After incubation for about 24 h, the reaction solution is examined by SDS-PAGE for cleavage (of the second part of the autoprotease from the protein of interest) having taken place. A Western blot is carried out to identify the proportions of processed (the second part of the N^{pro} autoprotease has been cleaved off from the fusion protein thereby liberating the protein of interest) and unprocessed (the second part of the N^{pro} autoprotease is still fused to the protein of interest) fusion protein. The proportion of cleaved material can be determined by densitometric analysis of the Coomassie-stained SDS-PAGE gel.

As used herein the term "inclusion bodies" shall refer to insoluble aggregates containing heterologous polypeptides (the fusion protein according to the present invention comprising the second part of the autoprotease fused to the protein of interest; of course, also the first part of the autoprotease can be expressed in inclusion bodies) present in the cytoplasm of transformed host cells. These appear as bright spots under the microscope and can be recovered by separation of the cytoplasm. The inclusion bodies are usually solubilised using a chaotropic agent. Upon solubilisation inclusion bodies are dissolved and a monomolecular suspension with substantially reduced intra- and inter-molecular interactions is aimed to obtain. Preferred solvents are urea, guanidine HCl and strong ionic detergents as N-lauroylsarcosine. In another embodiment of the present invention inclusion bodies are also solubilised using an aqueous alcohol solution at alkaline pH or simply an aqueous solution at alkaline pH.

As used herein, the term "recovering" shall refer to the obtaining of the protein of interest in purified form, i.e. by essentially separating the protein of interest from other proteins present in the expression/processing system, especially any (fragments) of the autoprotease, but also all other proteins or other components which should not be present in the intermediate or final product. The protein of interest recovered by the method according to the present invention may be commercialised in the bulk form obtained directly from the cleaving step according to the present invention or further purified and/or formulated into a specific formulation, e.g. into a pharmaceutical formulation.

As used herein the term "solubilisation" shall refer to the process necessary to dissolve the inclusion bodies. Solubilisation is aimed to result in a monomolecular dispersion of the polypeptides with minimum intra- and inter-molecular interactions. A preferred way of solubilisation of inclusion bodies within the scope of the present invention, is conducted by suspension in 50 mM Tris/HCl, 8 M urea, pH 7.3, adding a reducing agent, e.g. 50 mM DTT, in the case that oxidized cysteine residues are present. Where necessary it is possible to remove potentially insoluble material, for example by centrifugation. In the case that the inactive fusion protein is produced soluble within the cell, the clarified cell homogenate is subjected to the further work up described below for the solubilized inclusion bodies.

This technology is suited for a large variety of proteins of interest. For the purpose of the present invention, the terms "heterologous protein", "target protein", "polypeptide of interest" or "protein of interest" (and the like) mean a polypeptide which is not naturally cleaved by an autoprotease N^{pro} of a Pestivirus from a naturally occurring fusion protein or polyprotein (i.e. a polypeptide being different than the naturally following amino acids 169ff of the Pestivirus polyprotein encoding the structural Protein C and subsequent viral proteins). Examples of such heterologous proteins of interest are industrial enzymes (process enzymes) or polypeptides with pharmaceutical, in particular human pharmaceutical, activity.

Examples of preferred proteins of interest with human pharmaceutical activity are cytokines such as interleukins, for example IL-6, interferons such as leukocyte interferons, for example interferon a2B, growth factors, in particular haemopoietic or wound-healing growth factors, such as G-CSF, erythropoietin, or IGF, hormones such as human growth hormone (hGH), antibodies or vaccines. Also very short polypeptides having only 5 to 30 amino acid residues can be produced as protein of interest by the present technology. The N^{pro} technology has specific advantages in an expression system making use of inclusion bodies, because the strong aggregation bias of the fused autoprotease facilitates the formation of inert inclusion bodies, almost independent of the fusion partner. Accordingly, almost any protein of interest is producible with the present system in high amounts and yields. Reports are e.g. available for expression of synthetic interferon-a1; toxic gyrase inhibitor CcdB, a short 16-residue model peptide termed pep6His (SVDKLAAALEHHHHHH (SEQ.ID.NO.12)), human proinsulin, synthetic double domain D of staphylococcal protein A (sSpA-D₂), keratin-associated protein 10-4 (KRTAP10-4), synthetic green fluorescent protein variant (sGFPmut3.1), synthetic inhibitorial peptide of senescence evasion factor with N-terminal cysteine (C-sSNEVi), synthetic inhibitorial peptide of senescence evasion factor with randomized amino acid sequence with C-terminal cysteine (sSNEVscr-C); recombinant human monocyte chemoattractant protein 1 (rhMCP-1). So far, the only limitations with respect to high yields have been suspected for chaperones and proteins with comparable properties of supporting protein folding. Such proteins as fusion partners could suppress the aggregation bias of an N^{pro} molecule, leading to lower yields due to less aggregation. Nevertheless, the present technology can even be applied for expressing such proteins counter-acting aggregation.

The fusion protein according to the present invention can additionally contain auxiliary sequences, such as affinity tags or refolding aid moieties; it may also contain more than one protein of interest (it can e.g. contain two or three or four or even more proteins of interest which may be separated from each other at a later stage or even at the same stage as the cleavage by the Npro autoprotease).

With the present invention, an improvement is introduced into N^{pro} technology insofar that not the complete fusion protein consisting of N^{pro} autoprotease fused to a protein of interest has to be expressed in the expression system for producing the protein of interest, but that the first part of the N^{pro} moiety exhibiting the proteolytic activity is produced (e.g. expressed) separately so that autoproteolytic function can be selectively initiated by combining the first part with the second part of the autoprotease (the latter being fused to the protein of interest). Since the fusion protein according to the present invention (comprising the second part of N^{pro} autoprotease and the protein of interest) is smaller than the fusion protein in classical N^{pro} autoprotease technology (wherein the whole N^{pro} autoprotease (including the first part) is expressed), and therefore easier to express in recombinant production systems and easier to handle in processing before the cleaving step, the present invention allows higher titers and better productivity with respect to the protein of interest. Combination of the first (proteolytic) part and the second part (with the protein of interest) leads to complementation of the N^{pro} autoprotease, whereafter the autoproteolytic activity can act for cleaving off the protein of interest from the second part of the N^{pro} molecule.

As already mentioned, N-terminal autoprotease N^{pro} from Classical Swine Fever Virus (CSFV) can be used as an excellent tool for expression of recombinant proteins, especially in E. coli. Due to its autocatalytic cleavage it enables synthesis of proteins with an authentic N-terminus which is especially important for pharmaceutical applications. Furthermore, not only large proteins ("proteins of interest") but also small peptides can be stably expressed by C-terminal linking to N^{pro}. A high expression rate forces the fusion protein into inclusion bodies. After purification, N^{pro} is refolded and cleaves itself off after complementation according to the present invention.

N^{pro} is advantageous compared to intein technologies especially concerning its fusion partners, which can be cleaved off. Similar to split inteins, N^{pro} can be split up into two fragments, for example one ("first") ranging from amino acid 22-106 and the other ("second") part ranging from amino acid 113-168. It is essential that the second (C-terminal) part alone does not already lead to autoproteolytic cleavage of the fusion protein, but that cleavage ("liberation" of the protein of interest only occurs upon effective complementation with the first part of the N^{pro} autoprotease. By adding the first (N-terminal) part of N^{pro} a functional protease is formed by complementation and the fusion partner is cleaved off.

It is essential that the protein of interest to be produced by the present invention is attached C-terminally after Cys168 of the N^{pro} autoprotease, because this is the cleavage site where the peptidic bond between the C-terminus of the N^{pro} moiety (at Cys168) and the protein of interest is cleaved upon complementation in step (b) according to the present invention.

In comparison with the intein system, the present N^{pro} complementation system according to the present invention is superior over intein technologies. A major advantage over the intein-system is, that with the split Npro technique it is possible to produce peptides/proteins with authentic homogeneous N-termini (meaning a protein product in which all molecules have the same N-terminus; standard protein production in E. coli can lead to a mixture of fMet, Met, and correct N-terminus which is especially important for biopharmaceuticals to guarantee the authentic activity and to avoid adverse drug reactions elicited by non-natural N-termini. Especially if one N^{pro} fragment is bound to a solid support and the other part of N^{pro} is added, the proteolytic activity is triggered by the complementation on the solid support and the fusion protein is cleaved off. The division of N^{pro} into two fragments offers many applications in addition to a complementation system. The site at which Npro is subdivided can be used to integrate new features, such as an affinity tag. For example, 6 consecutive His residues can be introduced at positions 107 to 112 instead of the original sequence, thereby creating a His-tag. The resulting N^{pro} autoprotease still has proteolytic activity but the His-tag can be used for affinity chromatography. As another example, chaperone-like structures can be introduced into the autoprotease molecule, e.g. SlyD. SlyD is a member of the FKBP family and fulfils many duties in E. coli. The flap (IF) domain of SlyD has a chaperone-like activity with a high affinity to unfolded proteins. SlyD also contains a C-terminal stretch with many His residues which serves as a nickel storage and nickel supplier for the formation of Ni-Fe clusters. This nickel ion binding also regulates peptidyl prolyl isomerase (PPI) activity, which is responsible for accelerating the rate limiting trans to cis isomeration in protein folding. N^{pro} contains many Pro residues which could impair refolding. SlyD improves the folding of Npro in combination with its PPI activity. The nickel binding site can be used as an affinity tag for purification. The IF domain can ideally be integrated between the N-terminal and C-terminal fragment of N^{pro} due to the near vicinity to its N- and C-terminus. This adds a chaperone activity to N^{pro}.

Accordingly, in a preferred embodiment of the present method, the first and/or second part of the N^{pro} autoprotease were generated in a recombinant production system, preferably in a prokaryotic host cell, especially in E. coli host cells. Preferably, both, the first and the second part are generated in a recombinant production system. This can be performed in one or two production systems: If produced in one production systems, both, the first and the second part are generated in the same recombinant production system. Producing the parts in separate recombinant production systems may be advantageous with respect to flexibility; production in the same recombinant system may be advantageous with respect to production facilities. It is also possible to produce both parts in inclusion bodies or only one of them or none. Refolding can then be adapted in a flexible manner, due to the systems.

According to a preferred embodiment, at least the second part (including the protein of interest) is generated as inclusion bodies, then solubilised and brought into contact with the first part (in step (b) according to the present invention). The first part can also be generated in inclusion bodies and may or may not be allowed to refold before contact with the second part. In this preferred embodiment, refolding of the second part can e.g. be achieved simultaneously in step (b) of the present invention.

In general, the sequence of providing the two polypeptides according to the present invention as inclusion bodies, solubilising, refolding and performing step (b) of the present invention may, however, be adapted to the nature and physicochemical behaviour of the polypeptides in each individual case. For example, the polypeptides may be mixed already at an early stage (or even be present in the same expression system as inclusion body), or not contacted with each other until performance of step (b) according to the present invention. It follows e.g. that the steps of solubilising or refolding can be performed with the polypeptides mixed with each other or being conducted separately; also conditions can be applied which allow solubilisation/refolding of only one of the two parts (even being already mixed) where the other part does not (yet) solubilise/refold. Step (b) may then be initiated by applying suitable conditions which allow complementation and cleavage to cleave the protein of interest from the second part of the N^{pro} autoprotease.

Preferred examples for the first part of the N^{pro} autoprotease are deletion mutants of naturally occurring versions of the N^{pro} autoprotease. Such deletions, of corse, must not lead to inactivation of proteolytic activity. For example, amino acids 2 to 21 (the amino acid numbering follows the numbering of most naturally occurring N^{pro} autoprotease sequences of CSFV, such as listed in Becher et al., J. Gen. Virol. 78 (1997), 1357-1366) can be deleted without affecting proteolytic activity. It is therefore preferred to use a first part of the N^{pro} autoprotease lacking amino acids 2 to 21 ("Δ21 N^{pro}"). These preferred first fragments with proteolytic activity therefore start with the Met + GluPro motif (at positions 1 and 22/23), preferably followed by a (Val/Leu)(Tyr/Phe) motif (amino acids 24 and 25 of N^{pro}). Another example for possible deletion without affecting proteolytic activity is amino acids 148 to 150 (e.g. ThrProArg in "EDDIE" (Achmüller et al., 2007) or GluProArg in the Alfort sequence (Becher et al., 1997)).

According to the present invention, further deletions are possible, even if the proteolytic activity is slightly reduced, e.g. amino acids 26 to 30, 76/77, 108 to 110, 147 and 151.

However, significant reduction in proteolytic activity has to be prevented. Therefore, preferably, the following amino acids should not be deleted (in the first/second part):
- the ProSerGlu motif at amino acids 35 to 37 (also present in variations, such as ProSerAsp, ProSerPro, ProHisPro, etc.);
- the LeuProHis motif (amino acids 47 to 49 of N^{pro}), optionally with the following amino acid (amino acids 50);
- amino acids 54 to 60 (although here, sequence variations may lead to improved proteolytic activity (see WO 2006/113957 A; Achmüller et al., 2007));
- amino acids 83 to 91 and 94/95; and
- amino acids 158 to 160.

Sequence variations occur between the various natural isolates (see e.g. GenBank or EMBL databases); also selected mutations have been provided with improved properties (see WO 2006/113957 A; Achmüller et al., 2007; Achmüller, PhD thesis, March 2006, University of Innsbruck (AT)): For example,
- Cys112, Cys134 and Cys138 can be replaced by another amino acid residue, preferably Glu;
- His5, Lys16, Asn35, Arg53, Gly54, Arg57, Leu143, Lys145 and/or Arg150 can be replaced by another amino acid residue, preferably arginine (R) 53 with glutamic acid (E), glycine (G) 54 with aspartic acid (D), arginine (R) 57 with glutamic acid (E), and/or leucine (L) 143 with glutamine (Q);
- Val24, Ala27, Leu32, Gly54, Leu75, Ala109, Val114, Val121, Leu143, Ile155 and/or Phe158 can be replaced by another amino acid residue, preferably threonine or serine, especially alanine (A) 109, valine (V) 114, isoleucine (I) 155 and/or phenylalanine (F)158;
- Ala28, Ser71 and/or Arg150 can be replaced by another amino acid residue, preferably glutamic acid (E), phenylalanine (F) and/or with histidine (H), especially alanine (A) 28 can be replaced with glutamic acid (E), serine (S) 71 can be replaced with phenylalanine (F) and arginine (R) 150 can be replaced with histidine (H).

A preferred embodiment is the N-terminal protease of Pestivirus strain D32/00_HoBi (GenBank Accession No. AAS68353.1) or the variant Δ21N^{pro} (HoBi) (SEQ.ID.NO.13) thereof having a deletion of the amino acids 2 to 21 at the N-terminus and the "TSC" motif at the C-terminus instead of the naturally occurring "ASC" motif.

According to a preferred embodiment, the first part of the N^{pro} autoprotease comprises amino acid 22 to 30 of N^{pro} autoprotease, preferably amino acid 22 to 75, more preferred amino acid 22 to 112, 22 to 106, 1 to 106, 1 to 75 or 1 to 30, especially amino acid 1 to 112.

According to a preferred embodiment, the second part of the N^{pro} autoprotease comprises amino acid 31 to 168 of N^{pro} autoprotease, preferably amino acid 76 to 168, especially amino acid 113 to 168 and 107 to 168.

As already mentioned, the exact sequence and very nature of the autoprotease which is split and complemented in the method according to the present invention is not critical; accordingly, N^{pro} autoproteases already successfully used in the N^{pro} autoprotease technology represent preferred embodiments of the present invention. Preferred autoproteases can be chosen also according to the fusion partner ("protein of interest"). For example, preferred sequences are the N^{pro} sequences disclosed in WO 2006/113957 A (as SEQ.ID.NOs. 1-5, 32/33, 92-98, especially SEQ.ID.NO 5 ("EDDIE")).

The complementation step (b) can be carried out in solution; however, according to a preferred embodiment, either the first or the second part can be immobilised on a solid support before complementation. Complementation can then be initiated by adding the other part whereafter the proteolytic activity is activated by complementation on the solid support. The cleaved part (either the C-terminal (i.e. the second part of the) autoprotease (if the protein of interest moiety is coupled to the solid support) or the protein of interest (if an autoprotease moiety is coupled to the solid support)) is then released from the solid support and can be easily recovered. Therefore, according to a preferred embodiment of the present method, the first or the second part of the N^{pro} autoprotease (the latter being coupled to the protein of interest) is provided in immobilised form on a solid support and contacted with the second or first part of the N^{pro} autoprotease in step (b) so that the autoproteolytically active N^{pro} autoprotease is formed on the solid support. Immobilisation to the solid support is preferably effected by adsorption or covalent binding.

As solid phase material, all materials already applied in the present field are appropriate. Preferably, the solid phase is selected from the group consisting of chromatography material, especially supports based on cellulose, agarose, acrylamide, poly(styrene-divinylbenzene) or ethylene glycol-methacrylate copolymers, microtiter plates, nitrocellulose membranes, microchips, glass plates, or metal coated supports.

According to the present invention various types of solid phase supports may be used, such as the supports based on cellulose, agarose (Sepharose or Macro-Prep gels), dextran (Sephadex gels), acrylamide (Sephacryl, Trisacryl gels), silica (TSK, SW gels), poly(styrene-divinylbenzene) (Source or Poros gels), ethylene glycol-methacrylate copolymers (Toyopearl HW, TSK, PW, fractogel EMD gels) or mixtures, in particular of agarose and dextran (Superdex gel). The supports approved for human or veterinary use by the competent American authorities

(FDA for food and drug administration) or the European Union agencies will be more particularly selected. In addition, the support selected can be bonded, preferably by covalent bonding, to an affinity ligand (the support is said to be functionalized). The solid phase matrix may comprise, as the matrix backbone, any natural or synthetic and organic or inorganic material known per se to be applicable in solid phase separation of proteins and other biomolecules, e.g. natural or synthetic polysaccharides such as agar-agar and agaroses; celluloses, cellulose ethers such as hydroxypropyl cellulose, carboxymethyl celluose; starches; gums such as guar gum, and gum arabic, gum ghatti, gum tragacanth, locust bean gum, xanthan gum; pectins; mucins; dextrans; chitins; chitosans; alginates; carrageenans; heparins; gelatins; synthetic polymers such as polyamides such as polyacrylamides and polymethacrylamides; polyimides; polyesters; polyethers; polymeric vinyl compounds such as polyvinylalcohols and polystyrenes; polyalkenes; inorganic materials such as silicious materials such as silicon dioxide including amorphous silica and quartz; silicas; metal silicates, controlled pore glasses and ceramics; metal oxides and sulfides, or combinations of these natural or synthetic and organic or inorganic materials.

The matrix backbone is preferably selected from agar-agar, agaroses, celluloses, cellulose ethers such as hydroxypropyl cellulose, carboxymethyl cellulose, polyamides such as poly(meth)acryl-amides, polyvinylalcohols, silicas, and controlled pore glasses.

Especially interesting solid phase materials as matrix backbones are e.g. agar or agarose beads such as Sepharose and Superose beads from Pharmacia Biotech, Sweden and Biogel A from Biorad, USA; dextran based beads such as Sephadex, Pharmacia Biotech; cellulose based beads and membranes such as Perloza cellulose from Secheza, Czechoslovakia; composite beads such as Sephacryl and Superdex, Pharmacia Biotech; beads of synthetic organic polymers such as Fractogel from Toso-Haas, USA; POROS media from Perceptive Biosystems, USA, Bio-Rex, Bio-Gel P and Macro Prep from Biorad, HEMA and Separon from TESSEK and Hyper D and Trisacryl media from BioSepra, USA, Enzacryl and Azlactone, 3M, USA; beads of siliceous materials such as controlled pore glass, PROSEP, from Bioprocesing, England and Spherocil, BioSepra; and coated silica composites in the form of beads or membranes such as ACTI-DISK, ACTI-MOD and CycloSep from Arbor Technologies, USA.

Typically, the solid phase matrix backbone, as well as the resulting functionalised solid phase matrix, may, e.g., be in the form of irregular particles or spherical beads, membranes or sheets, moulded surfaces, or sticks. The solid phase material may further be fully or partly permeable or completely impermeable to proteins. In a particularly interesting embodiment of the present invention, the matrix is in the form of irregular or spherical beads with sizes in the range of 1-10000 µm, preferably 10-1000 µm; such as 10-60 µm for high performance applications and such as 50-500 µm, preferably 50-300 µm, for preparative purposes. Preferred materials (polymethacrylate monoliths) for use in the present invention are disclosed in Junbauer et al. (J. Chromatogr. A 1184 (2008), 62-79).

A particular interesting form of matrix is a density controlled matrix in the form of a conglomerate comprising density controlling particles. These conglomerates are especially applicable in large scale operations for fluidised or expanded bed chromatography as well as different batch-wise chromatography techniques in non-packed columns, e.g. simple batch adsorption in stirred tanks.

Affinity ligands for the fusion protein according to the present invention may be attached to the solid phase material by any type of covalent bond known per se to be applicable for this purpose, either by a direct chemical reaction between the affinity ligand and the solid phase material or by a preceding activation of the solid phase material or of the ligand with a suitable reagent known per se making it possible to link the matrix backbone and the ligand. Examples of such suitable activating reagents are epichlorohydrin, epibromohydrin, allyl-glycidylether; bis-epoxides such as butanedioldiglycidylether; halogen-substituted aliphatic compounds such as di-chloro-propanol, divinyl sulfone; carbonyldiimidazole; aldehydes such as glutaric dialdehyde; quinones; cyanogen bromide; periodates such as sodium-meta-periodate; carbodiimides; chloro-triazines such as cyanuric chloride; sulfonyl chlorides such as tosyl chlorides and tresyl chlorides; N-hydroxy succinimides; 2-fluoro-1-methylpyridinium toluene-4-sulfonates; oxazolones; maleimides; pyridyl disulfides; and hydrazides. Among these, the activating reagents leaving a spacer group SP1 different from a single bond, e.g. epichlorohydrin, epibromohydrin, allyl-glycidylether; bis-epoxides; halogen-substituted aliphatic compounds; divinyl sulfone; aldehydes; quinones; cyanogen bromide; chloro-triazines; oxazolones; maleimides; pyridyl disulfides; and hydrazides, are preferred.

Especially interesting activating reagents are believed to be epoxy-compounds such as epichlorohydrin, allyl-glycidylether and butanedioldiglycidylether.

For peptide affinity chromatography within the scope of the present invention, any matrix useful for the immobilization of peptide ligands can be used. Preferably Fractogel epoxy (M), from Merck, Darmstadt, Germany) or equally preferred "monolithic chromatography medium" CIM-epoxy is used. The ligands can be immobilized either directly onto the chemically activated backbone of the chromatography matrix, or via a spacer or linker. In the latter case a spacer is coupled to the chromatographic matrix, said spacer is then chemically activated, in order to allow binding of the ligand. Preferably Fractogel epoxy matrices are used in combination with spacers.

In a particularly preferred embodiment of the present invention the spacer is generated by reaction of the chromatographic matrix with diaminodipropylamine (DADPA) and subsequent reaction with succinic anhydride (SA). The resulting terminal carboxy group on the spacer is chemically activated and preferably linked to a terminal amino-group. The ligand is immobilized on the matrix or on the spacer via a reactive group that it comprises. In the case of peptide ligands such reactive groups may be either the amino, carboxy or the sulfhydryl group. Within the present invention anchorage of the peptide on the matrix or the spacer via an amino bond is particularly preferred.

Preferably, the solid phase used according to the present invention is provided as affinity chromatography material, and exhibits oligopeptide ligands as disclosed in WO 2006/113958 A2.

Preferably the solid support is a chromatography column. In principle any chromatography material capable of selectively binding the first part of N^{pro} or the fusion polypeptide (comprising the second part of N^{pro} and the protein of interest) can be used within the framework of the present invention. The matrix of the chromatography may, in a preferred embodiment, be in the form of a column, however, it may also be in other forms, like beads or organic materials like polyethylene glycol modified with an affinity peptide.

Chromatography materials suitable for use within the present invention may be based on a cellulose binding domain, they may be cation exchange chromatography materials using polycationic tags like e.g. polyarginine or polylysine as well as anion exchange chromatography with polyanionic tags like e.g. polyasparagine. Accordingly within the present invention the use of an affinity chromatography material as solid support is preferably selected from the group consisting of immobilized metal ion chromatography (IMAC), cation exchange chromatography, anion exchange chromatography, cellulose binding domain chromatography and peptide affinity chromatography.

More preferably the affinity chromatography used is cation exchange chromatography, wherein the fusion polypeptide comprises a polycationic tag. Even more preferred is the use of either a polyarginine or polylysine affinity tag.

For cation exchange chromatography the expressed polypeptides preferably comprise an N-terminal polycationic tag, for example a polyarginine or polylysine tag. The solution containing the expressed polypeptide that was extracted from the host cells is (filtered) and loaded onto a column packed with any medium suitable for cation exchange chromatography such as e.g. SP Sepharose FF, CM Sepharose FF, Fractogel EMD SO3-. Preferably buffers with low conductivity are applied. After loading unbound material may be washed out and refolding may be started by introduction of a buffer with low urea concentration. At a urea concentration lower than 0.5M the target protein is cleaved off and can be eluted from the column.

Another preferred embodiment of the present invention is one, wherein the solid support is an affinity chromatography or an anion exchange chromatography and wherein the polypeptide to be bound to the column comprises a polyanionic tag. More preferably, polyasparagine is used as affinity tag.

A further preferred embodiment to achieve the desired binding properties is immobilized metal ion affinity chromatography (IMAC). Accordingly, in a preferred embodiment of the present invention the solid support is immobilized metal ion affinity chromatography (IMAC) material, and the polypeptide to be bound (especially the fusion protein comprising the second part of Npro and the protein of interest) comprises a metal chelate affinity tag. In this case the polypeptide is detected and bound by means of a metal chelate affinity tag comprised in it. In a more preferred embodiment of the present invention, the metal chelate affinity tag is a polyhistidine affinity tag. IMAC is based on the specific coordinate covalent binding between histidine or other suitable unique amino acids (either naturally present on the surface of the protein or grafted with recombinant DNA techniques) and various immobilized metal ions, such as copper, nickel, zinc, or iron. Chromatographic materials known in the art for the use in IMAC may also be useful within the present invention. In a preferred embodiment of the present invention, Ni²⁺-Chelating Sepharose Fast flow (GE Healthcare, Uppsala, SE) is used as matrix.

Alternatively, the solid support may be an immunoaffinity chromatography material, employing epitope tags as described above which are present at the N-terminus of the polypeptide and are bound to the chromatographic matrix via an antibody recognizing said tag. Another preferred affinity chromatographic material is affinity chromatography using oligopeptide ligands as disclosed in WO 2006/113958 A.

A preferred embodiment of the present invention is **characterized in that** the first and/or the second part comprises additional moieties, preferably an affinity tag or a refolding aid moiety, especially a His-tag, SlyD (or parts of SlyD), oligo aminoacid stretches composed of either positive or negative charged moieties, Strep-tag and/or FLAG-tag.

The present method can in principle be applied for production of any protein of interest, especially for all proteins known to be producible by the N^{pro} autoprotease technique. Since the method according to the present invention is suitable for large-scale manufacturing and pharmaceutical good manufacturing practice, it is preferred to produce a protein for therapeutic use in humans with the present method, preferably a human recombinant protein or a vaccination antigen. Preferred proteins of interest therefore include the proteins referred to as the 151 recombinant pharmaceuticals approved for human use by the FDA or by the EMEA by 2009 in the review of Ferrer-Miralles et al. (Microbial Cell Factories 8 (2009), 17 doi:10.1186/1475-2859-8-17), especially the products based on proteins which have already been produced in an E. coli host cell: Dukoral (Oral cholera vaccine),Pegasys (Peginterferon alfa-2a), PegIntron (Peginterferon alfa-2b), Infergen (Interferon alfacon-1), Rebetron (Interferon alfa-2b), Roferon A (Interferon alfa-2a), Viraferon (Interferon alfa-2b), ViraferonPeg (Peginterferon alfa-2b), Intron A (Interferon alfa-2b), Beromun (Tasonermin), Actimmune (Interferon gamma-1b), IPLEX (Mecasermin rinfabate recombinant), Kepivance (Palifermin), Neulasta (Pegfilgrastim), Neumega (Oprelvekin), Neupogen (Filgrastim), Humalog (Insulin lispro), Humatrope (Somatotropin), Humulin (Human insulin), Insuman (Human insulin), Lantus (Insulin glargine), Fortical (Salmon calcitpnin), Apidra (Insulin glulisine), Exubera (Human insulin), Forcaltonin (Salmon calcitonin), Forsteo (Teriparatide), Forsteo/Forteo (Teriparatide), Genotropin (Somatotropin), Glucagon, Increlex (Mecasermin), Insulin human Winthrop (Insulin human), Norditropin (Somatropin), Nutropin (Somatropin), NutropinAQ (Somatropin), Optisulin (Insulin glargine), Preotact (Human parathyroid hormone), Protropin (Somatrem), Somavert (Pegvisomant), Betaferon/Betaseron (Interferon beta-1b), Lucentis (Ranibizumab), Natrecor (Nesiritide), Rapilysin/Retavas e (Reteplase), Ontak (Denileukin diftitox), Kineret (Anakinra), and Omnitrope (Somatropin).

Preferably, step (b) is performed in a buffer, especially a phosphate, hydrogen phosphate or Tris/HCl buffer. The buffer may also comprise salts, such as NaCl, polyols, such as glycerol or carbohydrates, or detergents, such as non-ionic detergents, anionic detergents, cationic detergents, zwitterionic detergents, non-detergent sulfobetains, e.g. cetyl trimethylammonium chloride (CTAC), sodium dodecyl sulfate (SDS), lithium dodecyl sulfate (LDS) or N-lauroyl sarcosine.

The conditions for step (b) can be optimised for the individual autoprotease by applying the knowledge of the N^{pro} technology as disclosed. For example, step (b) may be performed at a pH of 5 to 11, preferably at a pH of 6 to 9.5, especially at a pH from 6.5 to 8.5. This step (b) may be performed in the presence of a buffer comprising NaCl, preferably of 50 to 1000 mM NaCl, especially of 100 to 500 mM NaCl; phosphate ions and/or hydrogen phosphate ions, preferably 5 to 500 mM phosphate and/or hydrogen phosphate, especially 10 to 200 mM phosphate and/or hydrogen phosphate; glycerol, preferably 1 to 10 % glycerol, especially 2 to 8 % glycerol; Tris/HCl, preferably 1 mM to 5 M Tris/HCl, especially 5 mM to 2 M Tris/HCl. The buffer used in step (b) can further contain one or more of the following ingredients: L-arginine, low concentrations of denaturants, polyethylene glycol, low-molecular weight non-detergent zwitterionic agents such as sulfobetaines, substituted pyridines and pyrroles, and acid substituted aminocyclohexanes.

In a preferred embodiment, basic conditions are applied, especially in step (b), e.g. by the presence of more than 5 mM NaOH or KOH, preferably more than 25 mM NaOH or KOH, more preferred of more than 50 mM NaOH or KOH, especially more than 100mM NaOH or KOH (or mixtures of KOH and NaOH); or other basic components (or mixtures thereof) resulting in a pH of more than 10, especially of a pH from 11 to 14. A specifically preferred embodiment of step (b) applies denaturing conditions to the fusion protein, preferably by a mixture of urea guanidinium hydrochloride and NaOH.

The two parts of Npro as defined herein may be provided by established techniques, e.g. by way of inclusion bodies (especially the second part with the protein of interest). Inclusion bodies can be solubilised and refolded by such established techniques e.g. as disclosed in WO 01/11057 A, WO 01/11056 A, WO 2006/113957 A, WO 2006/113958 A, WO 2006/113959 A, and Achmüller et al., Nat. Meth. 4 (2007), 1037-1043. The solubilised/refolded molecules can then be applied to the complementing step. It is also possible to combine the solubilisation/refolding with the complementation step, preferably by dilution in the presence of the first part of the autoprotease or by solubilisation/refolding on a solid phase in the presence of the first part of the autoprotease.

According to another aspect, the present invention relates to an N^{pro} autoprotease fragment consisting of amino acid 22 to 30 of Npro autoprotease, preferably amino acid 22 to 75, more preferred amino acid 22 to 112, 1 to 75 or 1 to 30, especially amino acid 1 to 112.

Another aspect of the present invention relates to an N^{pro} autoprotease fragment part being fused by a peptidic bond to a protein of interest but not exhibiting a proteolytic activity, consisting of amino acid 31 to 168 of Npro autoprotease, preferably amino acid 76 to 168, especially amino acid 113 to 168.

The present invention also relates to an expression vector encoding for an N^{pro} autoprotease fragment (first part) or fusion protein (second part) according to the present invention. In the expression vector to be employed in the process according to the present invention, the fragment or fusion polypeptide is operably linked to at least one expression control sequence. Expression control sequences are, in particular, promoters (such as the lac, tac, T3, T7, trp, gac, vhb, lambda pL or phoA promoter), ribosome binding sites (for example natural ribosome binding sites which belong to the abovementioned promoters, cro or synthetic ribosome binding sites), or transcription terminators (for example rrnB T1T2 or bla).

The vector may also contain sequences encoding fusion domains, as described below, that are present at the N-terminal end of the fusion polypeptide and that are required for its binding to the affinity chromatography system, e.g. polyamino acids like polylysine or, for immunoaffinity chromatogography, so-called "epitope tags", which are usually short peptide sequences for which a specific antibody is available. Well known epitope tags for which specific monoclonal antibodies are readily available include FLAG, influenza virus haemagglutinin (HA), and c-myc tags.

In a preferred embodiment of the present invention, the expression vector is a plasmid.

Another aspect of the present invention relates to a host cell, preferably a prokaryotic host cell, especially an E. coli host cell, containing an expression vector according to the present invention. The transformed bacterial host cell, i.e. the expression strain, is cultivated in accordance with microbiological practice known per se. The host strain is generally brought up starting from a single colony on a nutrient medium, but it is also possible to employ cryo-preserved cell suspensions (cell banks). The strain is generally cultivated in a multistage process in order to obtain sufficient biomass for further use.

On a small scale, this can take place in shaken flasks, it being possible in most cases to employ a complex medium (for example LB broth). However, it is also possible to use defined media (for example citrate medium). Since in the preferred embodiment of the present invention it is intended that especially the expressed fusion polypeptide comprising the second part of N^{pro} is in the form of insoluble inclusion bodies, the culture will in these cases be carried out at relatively high temperature (for example 30°C or 37°C). Inducible systems are particularly suitable for producing inclusion bodies (for example with the trp, lac, tac or phoA promoter).

On a larger scale, the multistage system consists of a plurality of bioreactors (fermenters), it being preferred to employ defined nutrient media. In addition, it is possible greatly to increase biomass and product formation by metering in particular nutrients (fed batch). Otherwise, the process is analogous to the shaken flask. In the process according to the present invention, the inclusion bodies are isolated from the host cell in a manner known per se. For example, after the fermentation has taken place, the host cells are harvested by centrifugation, micro filtration, flocculation or a combination thereof, preferably by centrifugation. The wet cell mass is disintegrated by mechanical, chemical or physical means such as high pressure homogenizer, beads mills, French press, Hughes press, osmotic shock, detergents, enzymatic lysis or a combination thereof. Preferably, disruption of the cells takes place by high pressure homogenization. In the preferred embodiment where the recombinant fusion polypeptide is deposited as inclusion bodies, the inclusion bodies can be obtained for example by means of high-pressure dispersion or, preferably, by a simple centrifugation at low rotor speed. The inclusion bodies are separated by centrifugation or microfiltration or a combination thereof. The purity in relation to the desired polypeptide of interest can then be improved by multiple resuspension of the inclusion bodies in various buffers, for example in the presence of NaCl (for example 0.5 1.0 M) and/or detergent (for example Triton X 100). Preferably the purity of the inclusion body preparation is improved by several washing steps with various buffers (e.g. 0.5 % Deoxycholate followed by two times 1 M NaCl solution and finally distilled water). This usually results in removal of most of the foreign polypeptides from the inclusion bodies.

According to another aspect, the present invention relates to a solid support comprising a first part (i.e. an N-terminal part) of an N^{pro} autoprotease, wherein complementation of said first part with the C-terminal part (second part) of the N^{pro} autoprotease forms an autoproteolytically active N^{pro} autoprotease. This solid support is defined as mentioned above and comprises an N-terminal part of the N^{pro} autoprotease bound thereto. This N-terminal part of the N^{pro} autoprotease bound to the solid support can then be subjected to complementation according to the present invention. The binding of the N-terminal part of the N^{pro} autoprotease to the solid support is preferably effected by adsorption or covalent binding.

Alternatively, the present invention also relates to a solid support comprising a second part (i.e. a C-terminal part) of an N^{pro} autoprotease, said second part being fused by a peptidic bond to a protein of interest but not exhibiting a proteolytic activity (but being complementable with a first part of an N^{pro} autoprotease according to the present invention). This solid support is defined as mentioned above and comprises a C-terminal part of the N^{pro} autoprotease being fused by a peptidic bond to a protein of interest but not exhibiting a proteolytic activity bound thereto. This C-terminal part of the N^{pro} autoprotease bound to the solid support can then be subjected to complementation according to the present invention. Again, the binding of the C-terminal part of the N^{pro} autoprotease to the solid support is preferably effected by adsorption or covalent binding.

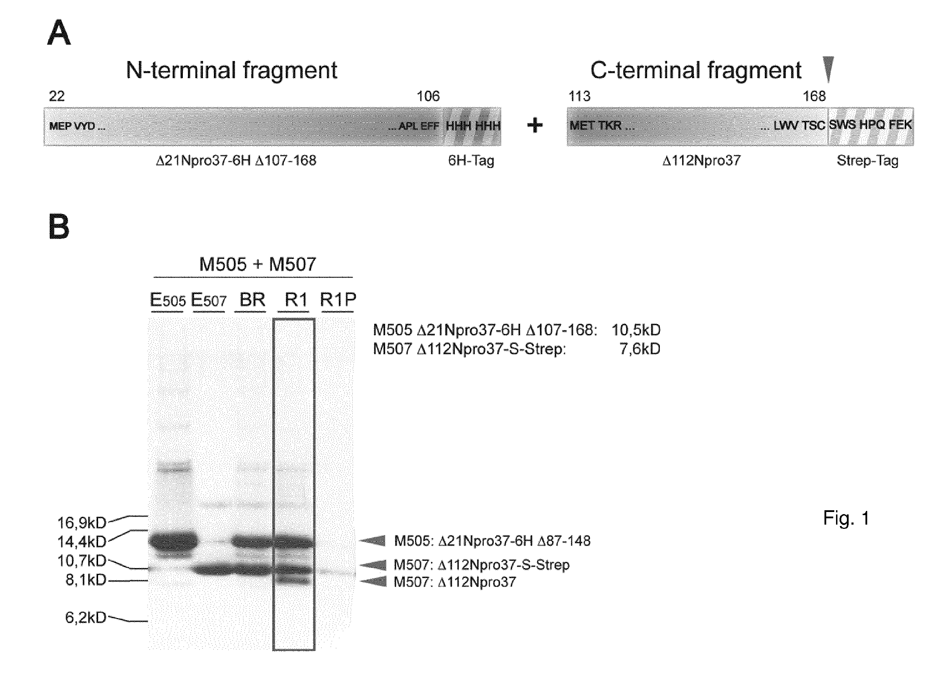

Figure 1: Complementation at position 112 with Npro fragments. A: scheme of N^{pro} fragments D21N^{pro}37-6H D107-168 (N^{pro}505) and D112N^{pro}37-S-Strep (N^{pro}507). B: Tris-tricine SDS-PAGE of the complementation at position 112. E505...eluted fraction of N^{pro}505; E507...eluted fraction of N^{pro}507; BR...before refolding; R...Refolding; RP...refolding pellet; Amount of protein in PAGE samples: E505: 23µg, E507: 17µg, BR: 35µg, R: 34µg.

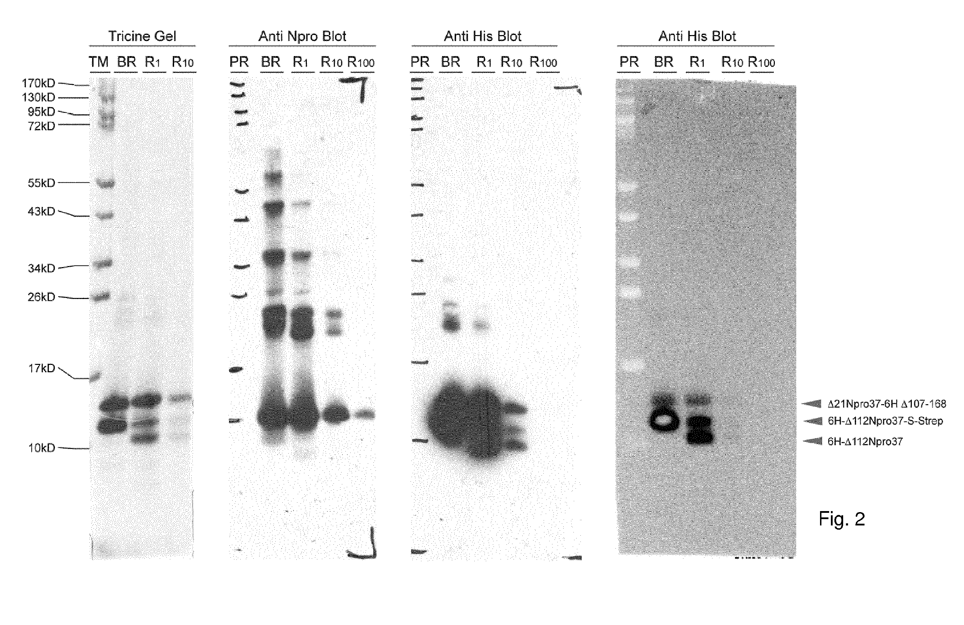

Figure 2: Western blot of the complementation of N^{pro}505 D21N^{pro}37-6H D107-168 (10, 5kD) with N^{pro}506 D92N^{pro}37-S-Strep (8, 3kD). BR...before refolding; R1...Refolding; R10...1:10 dilution of the refolding sample; R100...1:100 dilution of the refolding sample; Amount of protein in PAGE samples: BR: 62µg, R1: 62µg, R10: 6µg. Anti Npro Blot: anti-NproEDDIE (animal 10201) antibody, produced in goat, spec. IgG from 50ml antiserum (BioGenes GmbH) Anti His Blot: HisProbe HRP (Pierce). The left Anti His Blot was classically developed onto an X-ray film while for the right Anti His Blot an imager from Proxima was used.

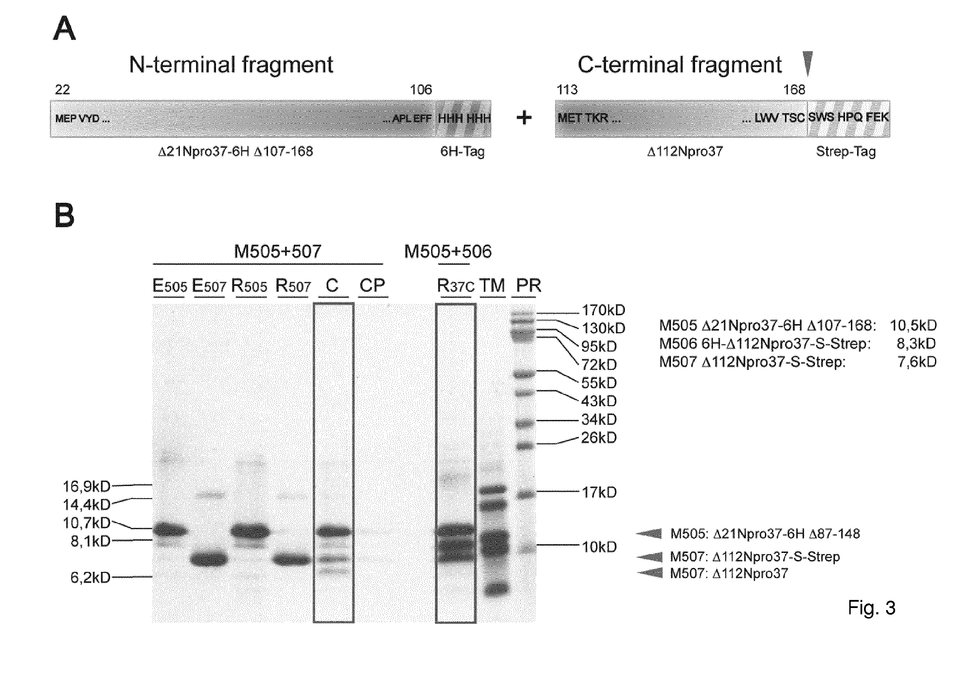

Figure 3: Complementation at position 112 with renaturated N^{pro} fragments. A: scheme of N^{pro} fragments D21N^{pro}37-6H D107-168 (N^{pro}505) and D112N^{pro}37-S-Strep (N^{pro}507). B: Tris-tricine SDS-PAGE of the complementation at position 112 with renaturated N^{pro} fragments. E₅₀₅...eluted fraction of N^{pro}505; E₅₀₇...eluted fraction of N^{pro}507; BR...before refolding; R...Refolding; RP...refolding pellet; R_{37C}...refolding at 37°C. Amount of protein in PAGE samples: E₅₀₅: 22µg, E₅₀₇: 14µg, R₅₀₅: 22µg, R₅₀₇: 14µg, R: 18µg.

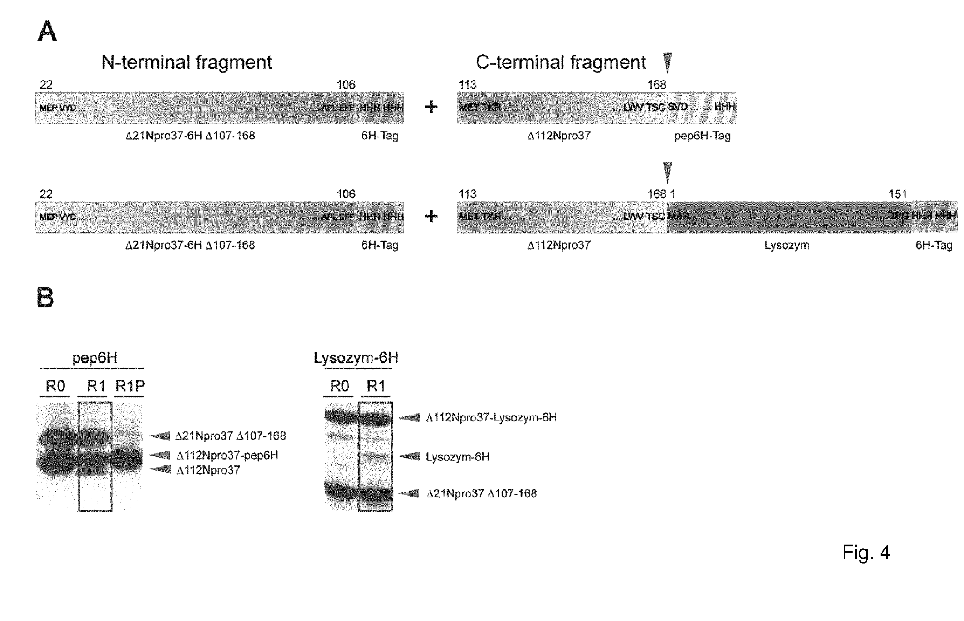

Figure 4: Complementation at amino acid position 112 with D112N^{pro}37-pep6H and D112N^{pro}37-Lysozyme-6H. A: scheme of D112N^{pro}37-pep6H and D112N^{pro}37-Lysozyme-6H. B: SDS-PAGE gels of the refoldings in different buffers.
R0...N^{pro} before refolding; R1...refolding after 1 day; R1P...Pellet of refolding after 1 day.

### Examples

### Materials and methods

### Preparation of fragments

DNA fragment coding for amino acid 22 to 106 of D21N^{pro}37 was amplified from a pET30a vector harboring D21N^{pro}37-pep6H with the forward primer 5'-GAAATTAATACGACTCACTATAGG-3' (SEQ.ID.NO.6), which has an NdeI restriction site and a reverse primer 5'-ATATAGTCGACTTATTAAAAGAATTCCAGAGG-3' (SEQ.ID.NO.7), which has a SalI restriction site as well as two TAA stop codons. Addition of a C-terminal 6H tag was achieved using the reverse primer 5'-ATAGTCGACTTATTAGTGATGGTGATGGTGATGAAAGAATTCCAGAGG-3' (SEQ.ID.NO.8). C-terminal fragments ranging from amino acid 113 to 168 in combination with a C-terminal Strep tag were generated without N-terminal 6H tag using forward primer 5'-TATACATATGGAAACCACTAAACGTATTGGC-3' (SEQ.ID.NO.9) and with 6H tag using forward primer 5'-TATACATATGCATCACCATCACCATCACGAAACCACTAAACGTATTGGC-3' (SEQ.ID.NO.10) and reverse primer 5'-GTTATGCTAGTTATTGCTCAGCGG-3' (SEQ.ID.NO.11). D21N^{pro}37-pep6H in a pET30a vector serves as template. The PCR products obtained were digested with NdeI and SalI, and ligated into a pET30a vector restricted with the same enzymes.

For expression of N^{pro} fragments *E. coli* strain BL21 (DE3) was used. *E. coli* BL21 (DE3) was transformed by electroporation and grown o/n at 37°C. After a 1:100 dilution, *E. coli* was grown until an OD₆₀₀ of about 0.5 was reached. Subsequently protein expression was induced by addition of 1mM IPTG to the suspension. After an incubation for 4 hours at 37°C, 225rpm the cells were harvested by centrifugation and lysis was achieved by French press treatment. IBs were collected by centrifugation.

N-terminal and C-terminal N^{pro} fragments were expressed separately. After solubilisation of IBs in a NaH₂PO₄ buffer containing 5M GdnHCl, 100mM NaCl, 10mM MTG, and 50mM NaH₂PO₄ buffer (pH 7.0) N^{pro} fragments were purified either by a Ni-NTA column or by a Strep-Tactin column depending on the fusion tag.

### Complementation during renaturation

About 1mg of the eluted N-terminal fragment D21N^{pro}37-6H D107-168 and 1mg of the C-terminal fragment D112N^{pro}37-S-Strep were pooled and refolded by 1:25 dilution in a NaH₂PO₄ buffer (pH 7.0) containing 30% glycerol, 100mM NaCl, 10mM MTG. After one day incubation at 20°C precipitated protein was pelleted by centrifugation at 5000g for 1 hour. A sample of the soluble fraction as well as from the pellet was loaded onto a denaturating Tris-Tricine PAGE gel (fig. 1).

### Complementation of renatured fragments

To make sure that the cleavage of N^{pro} is by complementation of two fragments and not by an intrinsic catalytic activity of the C-terminal fragment, another experiment was carried out. Therefore, the N-terminal fragment D21N^{pro}37-6H D107-168 and the C-terminal fragment D112N^{pro}37-S-Strep were expressed separately. After solubilisation of IBs, D21N^{pro}37-6H D107-168 was purified by a Ni-NTA column while D112N^{pro}37-S-Strep was purified by a Strep-Tactin resin. Eluted fractions containing N^{pro} were pooled. Then about 1mg of D21N^{pro}37-6H D107-168 and about 1mg of D112N^{pro}37-S-Strep were refolded by 1:25 dilution separately. After incubation at 20°C o/n both refoldings were combined and incubated a further day at 20°C. Afterwards precipitated protein was pelleted by centrifugation at 5000g for 1 hour. A sample of the soluble fraction as well as from the pellet was loaded onto a denaturating Tris-Tricine PAGE gel (fig. 2).

### Example 1: Complementation during renaturation

A denaturating Tris-Tricine PAGE of the complementation of denaturated N-terminal fragment D21N^{pro}37-6H D107-168 in combination with denaturated C-terminal fragment D112N^{pro}37-S-Strep shows that both parts complement each other and form a functional protease (fig. 1).

Western blots were conducted as a confirmation of the complementation experiments (fig. 2). It looks like the polyclonal anti N^{pro} antibody recognizes just the N-terminal fragment of N^{pro} while there is no signal from the C-terminal fragment. However, all three N^{pro} fragments (D21N^{pro}37-6H D107-168, 6H-D92N^{pro}37-S-Strep and 6H-D92N^{pro}37) could be identified by an India His blot. This verifies the operating principle of complementing two N^{pro} fragments.

### Example 2: Complementation of renatured fragments

A denaturating Tris-Tricine PAGE of the complementation of renatured N-terminal fragment D21N^{pro}37-6H D107-168 in combination with renatured C-terminal fragment D112N^{pro}37-S-Strep shows that the C-terminal fragment alone is not able to cleave off the Strep tag. Only by combining both parts of N^{pro} an active autoprotease is formed (fig. 3).

A further complementation was carried out at 37°C with fragments D21N^{pro}37-6H D107-168 and 6H-D112N^{pro}37-S-Strep. Both fragments were united in denaturating buffer and renaturated together by rapid 1:25 dilution (fig. 3). As the PAGE gel shows, the complementation works at 37°C as well.

### Example 3: Complementation with different C-terminal fusion partners

Further complementation experiments were carried out with different fusion partners coupled to the C-terminal fragment. A cleavage of the autoprotease was observed with D112N^{pro}37 in combination with pep6H and Lysozym-6H (fig. 4). Although the cleavage rate in combination with Lysozym-6H is very low, it shows that the principle of complementing two parts of N^{pro} works with other fusion partners than Strep.

### N^{pro} Sequences

D21N^{pro}37-6H D107-168 (N^{pro}505) (SEQ.ID.NO.1):

MEPVYDTAGR PLFGNPSEVH PQSTLKLPHD RGRGDIRTTL RDLPRKGDCR
SGNHLGPVSG IYIKPGPVYY QDYTGPVYHR APLEFFHHHH HH

6H-D112N^{pro}37-S-Strep (N^{pro}506) (SEQ.ID.NO.2) :

MHHHHHHETT KRIGRVTGSD GKLYHIYVEV DGEILLKLAK RGTPRTLKWT
RNTTNCPLWV TSCSWSHPQF EK

D112N^{pro}37-S-Strep (N^{pro}507) (SEQ.ID.NO.3) :

METTKRIGRV TGSDGKLYHI YVEVDGEILL KLAKRGTPRT LKWTRNTTNC
PLWVTSCSWS HPQFEK

D112N^{pro}37-pep6H (N^{pro}584) (SEQ.ID.NO.4) :

METTKRIGRV TGSDGKLYHI YVEVDGEILL KLAKRGTPRT LKWTRNTTNC
PLWVTSCSVD KLAAALEHHH HHH

D112N^{pro}37-Lysozym-6H (N^{pro}586) (SEQ.ID.NO.5) :

METTKRIGRV TGSDGKLYHI YVEVDGEILL KLAKRGTPRT LKWTRNTTNC
PLWVTSCMAR VQFKQRESTD AIFVHCSATK PSQNVGVREI RQWHKEQGWL
DVGYHFIIKR DGTVEAGRDE MAVGSHAKGY NHNSIGVCLV GGIDDKGKFD
ANFTPAQMQS LRSLLVTLLA KYEGAGLRAH HEVAPKACPS FDLKRWWEKN
ELVTSDRGHH HHHH

Δ21 N^{Pro} (HoBi) (SEQ.ID.NO.13)

MEPLYDKNGA VLFGEPSDTH PQSTLKLPHP RGEKEVIVGI RDLPRKGDCR
TGNRLGPVSG LFVKPGPVFY QDYSGPVYHR APLEQFKQAP MCEVTKRIGR
VTGSDGNLYH MYVCTDGCIL VKTAKREGQD VLKWVYNVLD SPIWVTSC

The SVDKLAAALEHHHHHH motif (SEQ.ID.NO.12) is a model peptide (with His-tag) attached to the autoprotease moiety.

## Claims

1. Method for producing a recombinant protein of interest **characterised by** the following steps:
(a) providing a first part of an N^{pro} autoprotease and providing a second part of an N^{pro} autoprotease, wherein said second part is fused by a peptidic bond to a protein of interest but said second part alone does not exhibit a proteolytic activity, and wherein complementation of said first part with the second part forms an autoproteolytically active N^{pro} autoprotease,
(b) contacting the first part of the N^{pro} autoprotease with the second part of the N^{pro} autoprotease so that an autoproteolytically active N^{pro} autoprotease is formed and the protein of interest fused by the peptidic bond to the second part of the N^{pro} autoprotease is proteolytically cleaved off the second part of the N^{pro} autoprotease at the peptidic bond, and
(c) recovering the protein of interest.

2. Method according to claim 1, **characterized in that** the first and/or second part of the N^{pro} autoprotease were generated in a recombinant production system, preferably in a prokaryotic host cell, especially in E. coli host cells.

3. Method according to claim 1 or 2, **characterized in that** the first part of the N^{pro} autoprotease comprises amino acid 22 to 30 of N^{pro} autoprotease, preferably amino acid 22 to 75, more preferred amino acid 22 to 112, 22 to 106, 1 to 106, 1 to 75 or 1 to 30, especially amino acid 1 to 112.

4. Method according to any one of claims 1 to 3, **characterized in that** the second part of the N^{pro} autoprotease comprises amino acid 31 to 168 of N^{pro} autoprotease, preferably amino acid 76 to 168, especially amino acid 113 to 168 and 107 to 168.

5. Method according to any one of claims 1 to 4, **characterized in that** either said first part or said second part of the N^{pro} autoprotease is provided in immobilised form on a solid support and contacted either with said second or said first part of the N^{pro} autoprotease, respectively, in step (b) so that the autoproteolytically active N^{pro} autoprotease is formed on the solid support.

6. Method according to any one of claims 1 to 5, **characterized in that** the first and/or the second part comprises additional moieties, preferably an affinity tag or a refolding aid moiety, especially a His-tag, SlyD, oligo aminoacid stretches composed of either positive or negative charged moieties, Strep-tag and/or, FLAG-tag.

7. Method according to any one of claims 1 to 6, **characterized in that** the protein of interest is a protein for therapeutic use in humans, preferably a human recombinant protein or a vaccination antigen.

8. Method according to any one of claims 1 to 7, **characterized in that** step (b) is performed at a pH of 5 to 11, preferably at a pH of 6 to 9.5, especially at a pH from 6.5 to 8.5.

9. N^{pro} autoprotease fragment consisting of amino acid 22 to 30 of Npro autoprotease, preferably amino acid 22 to 75, more preferred amino acid 22 to 112, 22 to 106, 1 to 106, 1 to 75 or 1 to 30, especially amino acid 1 to 112.

10. N^{pro} autoprotease fragment part being fused by a peptidic bond to a protein of interest but not exhibiting a proteolytic activity, consisting of amino acid 31 to 168 of Npro autoprotease, preferably amino acid 76 to 168, especially amino acid 113 to 168 and 107 to 168.

11. Expression vector encoding for an N^{pro} autoprotease fragment or fragment part according to claim 9 or 10.

12. Host cell, preferably a prokaryotic host cell, especially an E. coli host cell, containing an expression vector according to claim 11.

13. Solid support comprising an N-terminal part (first part) of an N^{pro} autoprotease, wherein complementation of said first part with the C-terminal part (second part) of the N^{pro} autoprotease forms an autoproteolytically active N^{pro} autoprotease.

14. Solid support comprising a C-terminal part (second part) of an N^{pro} autoprotease, said C-terminal part being fused by a peptidic bond to a protein of interest but not exhibiting a proteolytic activity but wherein complementation of the second part with the N-terminal (first) part of the N^{pro} autoprotease forms an autoproteolytically active N^{pro} autoprotease.
